Europäisches Patentamt

**European Patent Office**

Office européen des brevets

(11) Publication number: **0 211 659**
**A2**

(12) **EUROPEAN PATENT APPLICATION**

(21) Application number: **86306084.4**

(22) Date of filing: **06.08.86**

(51) Int. Cl.⁴: **B 01 D 53/26**
**C 10 K 1/16, C 07 C 9/04**
**C 01 B 3/00**

(30) Priority: **12.08.85 US 764365**

(43) Date of publication of application:
**25.02.87 Bulletin 87/9**

(84) Designated Contracting States:
**GB IT NL**

(71) Applicant: **THE DOW CHEMICAL COMPANY**
**2030 Dow Center Abbott Road P.O. Box 1967**
**Midland, MI 48640(US)**

(72) Inventor: **Robbins, Lanny A.**
**3900 Tittabawassee River Road**
**Midland Michigan 48640(US)**

(72) Inventor: **Weaver, Donald R.**
**4915 Tucker Street No. 3**
**Midland Michigan 48640(US)**

(74) Representative: **Burford, Anthony Frederick et al,**
**W.H. Beck, Greener & Co. 7 Stone Buildings Lincoln's Inn**
**London WC2A 3SZ(GB)**

(54) Gas drying process.

(57) Natural or industrial gas streams are dehydrated with a liquid organic desiccant and the desiccant is treated in a thermal reconcentration zone to remove a major portion of the water contained therein, followed by treatment in an isothermal azeotroping zone to remove substantially all the remaining water. The azeotroping agent is separated from the azeotrope and dried before being recycled to the azeotroping zone.

EP 0 211 659 A2

-1-

# GAS DRYING PROCESS

The present invention is directed to a process for dehydrating industrial gases. More specifically, the invention concerns a process for dehydrating a liquid organic desiccant and a process of using the dried desiccant to decrease the dew point of an industrial gas stream.

Industrial gases, such as hydrogen, natural gas and other normally gaseous hydrocarbons, often contain moisture from various sources. The moisture can cause problems, particularly in pipeline transportation of industrial gases, as it leads to hydrate formation. The hydrates are solids which lead to plugging of equipment. Thus, it is desirable to dry industrial gases to reduce moisture-related problems. The degree of dryness of a gas is indicated by its dew point. Accordingly, it is desirable to lower the dew point of industrial gases.

When gases, more particularly natural gas, are dried with a glycol, particularly triethylene glycol, water vapor is absorbed from the gas and a water-containing glycol is formed. If this glycol is to be reused for gas-drying purposes, the water-containing glycol must be reconcentrated to remove most of the water therefrom. Accordingly, the water-containing glycol is generally heated and the major part of the water is thereby vaporized and separated out, thereby forming a concentrated glycol of low water content. This reconcentrated glycol is returned into the gas-drying stage. Mere heating of the moist glycol cannot be used to accomplish complete drying of the glycol, since at maximum heating temperatures for glycols (about 205°C with triethylene glycol), a drying of only up to about 99.0 percent glycol is realized. When operating continuously, higher heating temperatures lead to a partial thermal decomposition of the glycol, so that the advantage of the higher reconcentration is lost due to increased expense for the replacement of the glycol.

Efforts have been made, however, to increase the concentration of the glycol still further, since the residual moisture of the gas dried with glycol proportionately decreases as the concentration of the glycol used for the drying is increased. It is thus possible, for example, when using a 99.9 percent triethylene glycol (instead of one which is only 99 percent) and a contact temperature with the gas to be dried of 20°C, to reduce the dew point of the gas by an additional amount of 20 to 40°C.

It is also known to cool natural gas with a content of liquid hydrocarbons in the dried or undried state down to temperatures of, for example, -30 to 0°C, and thereby to condense and separate out the liquid hydrocarbons and the moisture possibly contained in the gas. With undried natural gas, dried glycol can be sprayed as a hydrate inhibitor into the stream of natural gas prior to cooling and moist glycol and liquid hydrocarbon are separated out as condensate after the expansion of the natural gas to pipeline pressure. This moist glycol, like the moist glycol which is formed from drying of the natural gas by absorption with glycol, is again reconcentrated and is used for spraying or for absorption purposes.

It is also known to improve still further the degree of drying of the thermally reconcentrated glycol by stripping the glycol being reconcentrated with a heated dry gas. Serving as stripping gas is dried natural gas, which is heated in the reboiler and is then brought into contact with the preconcentrated glycol discharging from the reboiler (see U.S. Patent 3,105,748). One disadvantage of this process is the loss of part of the valuable product gas which is consumed for stripping purposes. The stripping gas charged with the water vapor is generally blown off into the atmosphere or burned. To avoid this loss, the stripping gas can also be recirculated, whereby the stripping gas is cooled and an aqueous phase is deposited in a separator. The stripping gas

-4-

as thus dried returns, together with supplementary gas, into the stripping section of the reconcentration plant (see U.S. Patent 3,867,112). While it is true that the consumption of stripping gas and the contamination of the air are smaller in this case, the expense for the working up and recirculation of the stripping gas is considerable.

For a given gas-drying process, a drier glycol will give a drier industrial gas. U.S. Patent 3,151,959 discloses a process which employs ion-exchange resins or zeolites (molecular sieves) to dry the glycol to a water content of less than 2 percent. The use of molecular sieves and other solid desiccants is disadvantageous in that they eventually become saturated. At that time, the solid desiccant must be regenerated. Thus, the solid desiccant process requires multiple beds of solid dessicants to operate continuously.

Hygroscopic liquids are widely used for drying numerous industrial gases, by passing the comparatively dry, liquid hygroscopic desiccant through a column in a direction counter to the flow of the gas. Dry gas is usually removed from the top of the column, while the rich liquid desiccant, containing the absorbed water, is removed from the bottom of the column and sent to a regenerating unit where the rich desiccant is heated to remove as much absorbed water as possible without excessively decomposing the desiccant. The so regenerated liquid desiccant is then recycled to the gas drying column.

The hygroscopic nature of the desiccants serves well for absorption of water, however, there are problems in their regeneration. With heat alone, a temperature approaching the boiling point of the desiccant, which frequently exceeds the decomposition range of the liquid desiccant, is needed to reduce the moisture content to a level satisfactory for the intended desiccant application. More serious, however, are the effects of direct contact of the desiccant with hot surfaces of a heat exchanger. It is known that excessive heat fluxes on such surfaces contribute greatly to break-down of the desiccants.

Previous attempts at solving the above problems have not been entirely satisfactory.

Gas stripping is well-known, i.e., it is well-known that liquid desiccants such as diethylene glycol can be dehydrated by treating the desiccant with some of the (natural) gas previously dried by the desiccant or with some other dry gas. (See, for example, U.S. Patents 3,105,748; 3,318,071; 3,348,601; 3,370,636; 3,397,731; 3,841,382; 3,867,112; 4,070,231; 4,179,328; 4,273,620; 4,280,867; and 4,314,891.)

Stripping with steam or naptha is also known. (See, e.g., U.S. Patents 4,162,145; 3,844,736; and 3,471,370). In a process described in U.S. Patent 3,471,370, naphtha and a glycolamine desiccant-acid gas absorbent are fed to a reboiler, situated externally of a stripping column, into which a moist glycolamine is fed. The temperature of the reboiler is maintained at 300-400°F (150-205°C). In the reboiler, all the naphtha, except that which may remain in solution in the absorbent at the high temperature, is flashed and the vapors are passed through the moisture stripping zone to dry the glycolamine mixture to a moisture content of 1.6 to 1.8 percent. In this procedure, the moisture content of the desiccant cannot be reduced below the concentration which is in equilibrium with the water content of the naphtha. It also has the shortcoming of exposure of the desiccant to hot surfaces.

In another procedure, described in U.S. Patent 3,349,544, an azeotroping agent is introduced below the surface of a liquid desiccant in a heated regeneration zone, while maintaining the temperature in the regenerating zone above the vaporization temperature of the azeotroping agent. The azeotropic mixture is condensed, water and azeotroping agent are separated and the azeotroping agent is recycled. In this process the moisture content of the desiccant cannot be reduced below the concentration which is in equilibrium with the water contained in the azeotroping agent.

U.S. Patent 4,009,083 discloses a process for regenerating liquid desiccants such that the regenerated desiccant contains from 1 to about 5000 ppm water (column 3, line 33.) The process comprises feeding a rich liquid absorbent into an upper portion of a regeneration column; vaporizing by heat a normally liquid hydrocarbon or mixture of hydrocarbons substantially insoluble in the dried desiccant and in water, which hydrocarbon has a boiling temperature below the upper and above the lower critical solution temperatures of the mixture of the desiccant and the said hydrocarbon under the pressure conditions employed; passing the vapors upwardly through the rich liquid absorbent, said vapors being the sole source of heat added for regeneration of the desiccant; condensing overhead vapors from the regenerator out of direct contact with the rich liquid absorbent; separating liquid hydrocarbon from water; and returning at least a portion of the overhead hydrocarbon condensate to the regeneration column as reflux; passing a two-phase liquid mixture from the regenerator into a separator to form a lean desiccant phase and a liquid hydrocarbon phase; passing the hydrocarbon phase into a vaporizer wherein the hydrocarbon phase is heated and vaporized for recycle to the regenerator; and removing the lean desiccant phase from the separator. The process is disadvantageous in that two liquid phases can exist in the regenerator, thus leading to potential surges or instability in operations.

Surprisingly, the present invention provides such an improved continuous process for drying industrial gases. The process is capable of producing dried gas having a dew point of about -95°C or less, and does not require the use of the dried gas to dry the desiccant.

Specifically, the present invention provides such an improved process, and involves the following steps:

(a) contacting a moisture laden gas stream with a substantially completely dehydrated liquid organic desiccant in a contacting zone to generate a gas stream having an increased dew point depression and a water--rich liquid organic desiccant;

(b) heating said water-rich liquid organic desiccant from said contactor zone in a thermal reconcentration zone heated to a temperature varying between the boiling point of said water-rich desiccant and the decomposition temperature of the pure desiccant whereby a major portion of the water is removed from said liquid desiccant;

(c) contacting said partially dried liquid desiccant with a hydrocarbon azeotroping agent which is substantially insoluble in said desiccant and which boils in the range from 35 to 235°C or mixtures thereof, in an azeotroping zone uniformly heated to a temperature in the range from 5 to 40°C below the decomposition temperature of the pure desiccant whereby substantially all the remaining water is removed from said liquid desiccant;

(d) recovering a water-hydrocarbon azeotrope;

(e) separating a hydrocarbon-rich phase from a water-rich phase;

(f) recycling said hydrocarbon-rich phase to said azeotroping zone;

(g) recovering a dehydrated organic desiccant; and

(h) recycling said dehydrated desiccant to said contacting zone;

the improvement comprises further drying the hydrocarbon azeotroping agent before it is recycled in step (f) to the azeotroping zone.

(See U.S. Patent 4,005,997 for its teachings regarding steps (a)-(h) of the present process.)

The process of the present invention can advantageously be employed to dry an industrial gas, such as, for example, hydrogen, methane, natural gas, ethylene, propylene, cracked gas, synthetic natural gas, and other normally gaseous hydrocarbons.

The liquid organic desiccant can be any liquid hygroscopic material. Representative desiccants include polyols alone or in mixture. Typical of these are ethylene glycol, propylene glycols, butylene glycols, pentylene glycols, glycerol, trimethylol propane, diethylene glycol, triethylene glycol, tetraethylene glycol, dipropylene glycol, tripropylene glycol, tetrapropylene glycol and mixtures thereof. These glycols preferably contain 2 to 12 carbon atoms. Other liquid desiccants that can be used in the present invention are polyols such as diglycerol, trimethylol propane, 2,2-dimethylol propane, 2,2,2-trimethylol ethane, 1,4-dihydroxy cyclohexane, 1,4-dimethylol cyclohexane, and mixtures thereof.

Polyol compounds which are normally solid, but which are soluble in substantially anhydrous liquid polyols or liquid hydroxyl amines, can also be included. Typical representatives of such solids are erythritol, sorbitol, pentaerythritol, and the low molecular weight sugars. Typical alkanolamines include monoethanolamine, diethanolamine, triethanolamine, isopropanolamine, including mono-, di- and triisopropanolamine or diglycolamine. Typically, the alkanolamines can contain from 2 to 9 carbon atoms.

Other desiccants include N-methyl pyrrolidone and alkyl-substituted derivatives thereof, sulfolane (tetramethylenesulfoxide) and alkyl-substituted derivates thereof. Mixtures of any of the above desiccants can be used in any proportion. Triethylene glycol is the preferred liquid desiccant. The liquid desiccant is contacted, in any known manner, with the gas to be dried so that the concentration of water in the gas is reduced.

The hydrocarbon azeotroping agents which can be employed in this invention are alkanes, cycloalkanes, and aromatic hydrocarbons which are substantially insoluble in the liquid desiccant and which have a normal boiling range from 35 to 235°C. Examples of the alkanes are those having straight and branched chains and which have 5-13 carbon atoms such as pentane, hexane, octane, as well as dodecane, isooctane (2,2,4-trimethylpentane), tridecane and mixtures thereof. Isooctane is the preferred azeotroping agent. Examples of the cycloalkanes are cyclohexane, dimethylcyclohexane, trimethylcyclohexane, cycloheptane, cyclooctane, and mixtures thereof. Examples of the aromatic hydrocarbons are benzene, toluene, xylene, ethylbenzene, cumene, butylbenzene, diethylbenzene, triethylbenzene, and mixtures thereof.

The azeotroping agent is added in an amount sufficient to substantially reduce or completely remove the water in the liquid desiccant. Azeotropic distillation is well-known [see, e.g., "Azeotropic Separation", Chemical Engineering Progress, 59(6), pp. 67-78 (June 1963)].

In the process of the present invention, the liquid desiccant is dried to lower water concentrations as compared to previous gas drying processes, such as that of U.S. Patent 4,005,997, which employed azeotropic distillation to dry the liquid desiccant. The drier liquid desiccant allows more complete gas drying. The improvement which leads to lower water concentrations is the addition of a drying column. The drying column serves to dry the hydrocarbon azeotroping agent. The drying column can be of any design which will lower the concentration of water in the azeotroping agent. Advantageously, the drying column requires very little energy relative to the total energy requirements of the entire process, which is very energy efficient compared to the gas stripping process. (See U.S. Patent 4,005,997, for a discussion of energy requirements in gas drying processes.) Preferably, the drying column is a distillation column. Examples of drying columns include beds of solid desiccants, such as molecular sieves or zeolites, crystallizers, strippers, and the like. The drying column can be operated in any manner which will dry the hydrocarbon azeotroping agent. For example, the use of distillation columns to remove water from liquid hydrocarbons is known. [See, e.g., "Drying Liquid Hydrocarbons Via Fractional Distillation", Chemical Engineering Progress, 66(8), pp. 54-60 (August 1970).]

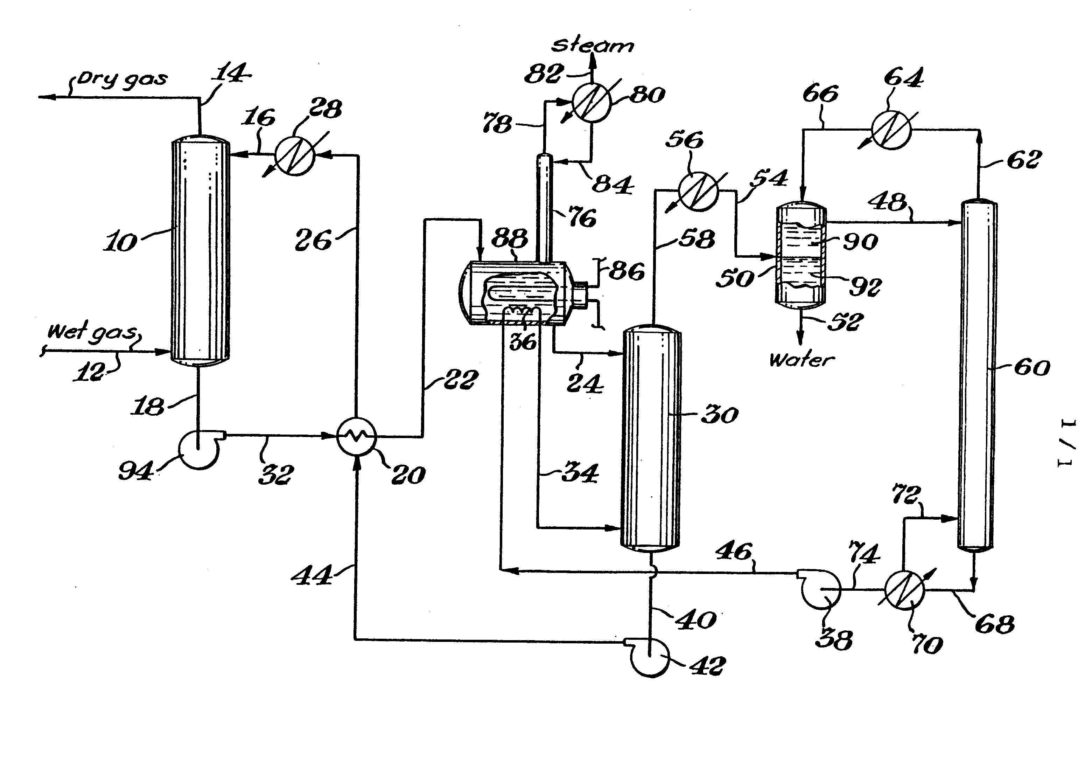

The accompanying figure is a diagrammatic view of one embodiment of the equipment which can be employed in the present invention. In the figure, the contactor 10 is a conventional gas-liquid contacting column having an inlet 12 for the wet industrial gas and an outlet 14 for the gas that has been dried. The previously dried or lean liquid desiccant enters the column 10 at the inlet 16 and the water-rich desiccant is removed from the outlet 18.

The rich desiccant is then pumped by pump 94 through line 32 to a feed-bottoms cross-exchanger 20 where it is heated to a temperature in the range of 80 to 180°C by the hot dry desiccant in line 44. The rich desiccant flows from the exchanger 20 through line 22 to a desiccant boiler 88 where the desiccant is initially dried. Heat for the boiler is provided by a fired-tube using fuel gas 86. Vaporized water is removed from the desiccant in tower 76 and passes via line 78 to partial condenser 80. The condensed portion is refluxed to tower 76 via line 84, while the noncondensed steam exits the condenser via line 82.

The initially dried desiccant having a water content of approximately 1.5 weight percent passes out the bottom of boiler 88 via line 24 and enters the uniformly heated azeotroping column 30 wherein substantially all the remaining water is removed. This column 30 is provided with heating coils and insulation jackets (not shown) which when combined with the hot azeotroping vapors passing through insure that the rich desiccant is treated to uniform temperatures in the range from 5 to 40°C below the decomposition temperature of the desiccant for a substantial contact period in which almost all the remainder of the water in the desiccant is removed. Preferably the azeotroping column and/or zone is uniformly heated in the range from 5 to 20°C below the decomposition temperature of the pure desiccant. Highest efficiencies are obtained at about 5°C below said temperature. Other means of heating such as electric coils can be used if it is convenient.

The hot dried desiccant passes out the bottom of the azeotroping column 30 by means of line 40 and is pumped by the pump 42 to the cross-exchanger 20 via line 44 and then via line 26 to a heat-exchanger 28 for further cooling. The dried desiccant then passes via line 16 to the contactor 10 for reuse.

The overhead azeotrope vapors leave the column 30 by line 58 to a conventional condensor 56 where they are converted to a liquid which then flows through line 54 to a separator 50. In the separator, the water-rich phase 92 is removed and discarded by line 52.

The wet liquid hydrocarbon-rich phase 90 (azeotroping agent) is fed by line 48 to hydrocarbon azeotroping agent drying column 60, wherein substantially all of the remaining water is removed. Wet azeotroping agent vapors pass out the top of drying column 60 via line 62 and are condensed in condenser 64. The condensed wet azeotroping agent then passes via line 66 to separator 50.

Dried azeotroping agent passes out the bottom of drying column 60 via line 68 to partial reboiler 70. The dry vapor from partial reboiler 70 is returned to the bottom of drying column 60 via line 72, while the dried liquid azeotroping agent is fed via line 74 to the pump 38 and hence to azeotrope vaporizer 36 by line 46 for reuse. In azeotrope vaporizer 36, the liquid azeotroping agent is converted to a vapor and this is supplied to the bottom of column 30 by line 34.

For the purposes of this invention the following definitions apply. "An azeotroping agent substantially insoluble in the liquid desiccant" means that the azeotroping agent is less than 5 percent by weight soluble in the desiccant and preferably less than 1 percent by weight soluble.

"A uniformly heated azeotroping zone" means that the temperature is maintained as constant as is thermodynamically possible throughout the zone, i.e., ±4°C from the designed temperature.

The following example is given to illustrate the invention and should not be construed as limiting its scope. All parts and percentages are by weight unless otherwise indicated.

Example 1

Referring again to the Figure, 100 x $10^6$ standard cubic feet ($2.8 \times 10^6$ $m^3$) per day of saturated natural gas containing 34 pounds (15.5 kg) of water per million standard cubic feet (SCF) ($28 \times 10^3$ $m^3$) of gas is fed to water absorber 10 via line 12. The water absorber 10 operates at 80°F (27°C) and 1000 psi (7 MPa).

Dried natural gas having a dew point of -145°F (-98 °C) and containing 0.001 pound (0.45 g of water per million SCF ($28 \times 10^3$ $m^3$) of gas, exits absorber 10 via line 14. The dew point depression of the gas is the difference between 80°F and -145°F, and is 225°F (125°C).

A mixture of triethylene glycol and water exits absorber 10 via line 18 and enters reboiler 88. Steam (118 lb/hr; 53.5 kg/hr) exits the top of the column 76 via line 82. Dried glycol containing 1.5 percent water passes via line 24 to azeotroping column 30, which is operated at 400°F (204 °C) and atmospheric pressure. Lean triethylene glycol containing 1.4 ppm water (i.e.,

99.99986 percent glycol) passes from the bottom of column 30 at a rate of 2,000 lb/hr (910 kg/hr) to the top of absorber 10 via lines 40, 44, 26 and 16.

Overhead azeotrope vapors pass via lines 58 and 54 to phase separator 50, which operates at 100°F (38°C). Separator 50 produces 24 lb (11 kg) water per hour through line 52, and 453 lb/hr (205 kg/hr) of a hydrocarbon-rich isooctane phase containing 220 ppm water. The isooctane phase enters drying column 60 via line 48. Column 60 operates at atmospheric pressure and 211°F (99.44°C) to produce an overhead which is 23 lb/hr (10.5 kg/hr) of isooctane containing 4400 ppm water. The overhead is recycled to separator 50 via lines 62 and 66. Dry isooctane containing 0.5 ppm water exits drying column 60 via line 74 at a rate of 430 lb/hr (195 kg/hr). Reboiler 70 operates at 30,000 BTU/hr (63 kJ /hr) and partially reboils the isooctane. The dry isooctane is returned to column 30 to dry additional glycol.

CLAIMS

1. A process for increasing the dew point depression of a gas using low energy requirements which has the steps of:

(a) contacting a moisture laden gas stream with a substantially completely dehydrated liquid organic desiccant in a contacting zone to generate a gas stream having an increased dew point depression and a water-rich liquid organic desiccant;

(b) heating said water-rich liquid organic desiccant from said contactor zone in a thermal reconcentration zone heated to a temperature between the boiling point of said water-rich desiccant and the decomposition temperature of the pure desiccant whereby a major portion of the water is removed from said liquid desiccant;

(c) contacting said partially dried liquid desiccant with a hydrocarbon azeotroping agent which is substantially insoluble in said desiccant and which boils in the range from 35 to 235°C or mixtures thereof, in an azeotroping zone uniformly heated to a temperature in the range from 5 to 40°C below the decomposition temperature of the pure desiccant whereby substantially all the remaining water is removed from said liquid desiccant;

(d) recovering a water-hydrocarbon azeotrope from said azeotroping zone;

(e) separating a hydrocarbon-rich phase of said azeotrope from a water-rich phase;

(f) recycling said hydrocarbon-rich phase to said azeotroping zone;

(g) recovering a dehydrated organic desiccant; and

(h) recycling said dehydrated desiccant to said contacting zone; characterised in that said hydrocarbon-rich phase is further dried prior to step (f).

2. A process as claimed in Claim 1, wherein said hydrocarbon-rich phase is dried using a solid material.

3. A process as claimed in Claim 2, wherein the solid material comprises a zeolite, a molecular sieve, or an ion-exchange resin.

4. A process as claimed in Claim 1, wherein said hydrocarbon-rich phase is dried using a distillation column.

5. A process as claimed in any one of the preceding claims, wherein the dried gas has a dew point of less than -95°C.

6. A process as claimed in any one of the preceding claims, wherein the dew point of the dried gas is at least 125°C lower than the dew point of the moisture laden gas.

7.    A process as claimed in any one of the preceding claims, wherein the azeotroping agent is isooctane.

8.    A process as claimed in Claim 7, wherein the dried isooctane contains less than about 1 ppm water.

9.    A process as claimed in any one of the preceding claims, wherein the gas stream comprises natural gas, and the liquid desiccant is a glycol having from 2 to 12 carbon atoms.

10.    A process as claimed in Claim 9, wherein the glycol is triethylene glycol and the azeotroping agent is isooctane.